# EUROPEAN PATENT SPECIFICATION

(11) **EP 1 562 952 B1**
(45) Date of publication and mention of the grant of the patent: **24.10.2007**
(21) Application number: 03783210.2
(22) Date of filing: 05.11.2003
(51) Int. Cl.: C07D 489/02

(54) **METHOD FOR THE CATALYTIC PRODUCTION OF HYDROCODONE AND HYDROMORPHONE**
KATALYTISCHES VERFAHREN ZUR HERSTELLUNG VON HYDROMORPHONE UND HYDROCODONE
PROCEDE DE PRODUCTION CATALYTIQUE D'HYDROCODONE ET D'HYDROMORPHONE

(30) Priority: 11.11.2002 US 425360 P
(43) Date of publication of application: 17.08.2005
(73) Proprietor: MALLINCKRODT INC., St. Louis, Missouri 63134 (US)
(72) Inventor: WANG, Peter, Xianqi, Chesterfield, MO 63017 (US); WHITE, Carl, Ray, St. Louis, MO 63122 (US)
(74) Representative: Cockerton, Bruce Roger
(86) International application number: PCT/US2003/035462
(87) International publication number: WO 2005/047291

(56) References cited:
- WO-A-98/05667
- US-A- 2 654 756
- NINAN, ALEYAMMA ET AL: "An improved synthesis of noroxymorphone" TETRAHEDRON , 48(32), 6709-16 CODEN: TETRAB; ISSN: 0040-4020, 1992, XP002285612

## Description

An aspect of the present invention is to provide a method for the catalytic conversion of a compound of Formula I into a compound of Formula II utilizing at least one transition metal complex of a tertiary phosphine halide as catalyst, 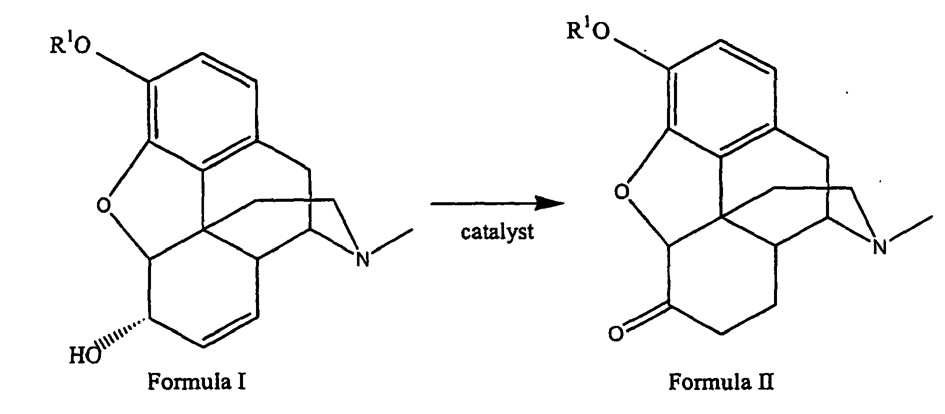 wherein R¹ is H, alkyl, aryl or acyl.

Another aspect of the present invention is to provide a one step method for the conversion of codeine or morphine into hydrocodone or hydromorphone, respectively.

### DETAILED DESCRIPTION

There is provided a method for the conversion of a compound according to Formula I to a compound according to Formula II, 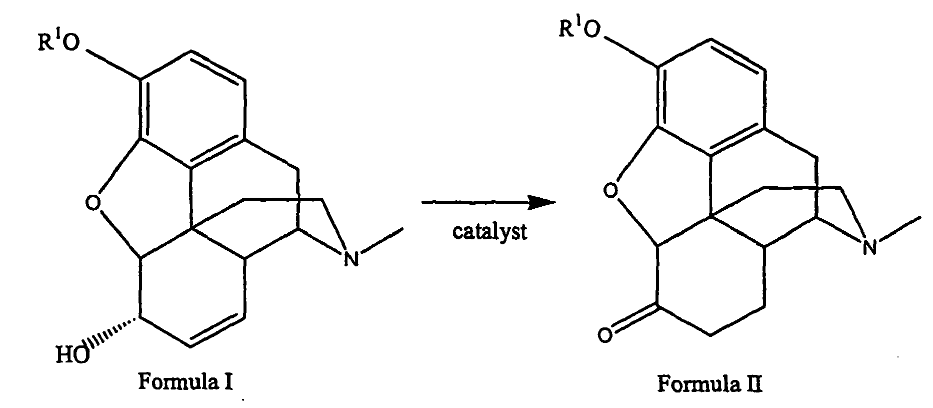 wherein R¹ is H, alkyl, aryl or acyl.

The method of the present invention is especially useful where R¹ is methyl or H, i.e., codeine or morphine, respectfully, leading to the formation of hydrocodone or hydromorphone, respectfully.

The transition metal catalysts of the present invention comprise a transition metal complex of a tertiary phosphine halide, [M(PR²₃)ₙX_{m]p,} wherein M is preferably a Group VIII transition metal, R² is an alkyl or aryl, X is a halide or halide compound, n is 1, 2, 3 or 4, and m is 1 or 2. These complexes are capable of polymerizing, therefore p is at least 1. Preferred metals include Rh, Ru, Pd and Pt. The halide, X, is typically Cl or Br. Halide compounds include but are not limited to BF₄, PF₆, ClO₄, CF₃SO₃ and OCOCF₃. R² is an alkyl or aryl group, with phenyl being preferred. Many of these catalysts are commercially available, or are easily prepared as is known in the art.

In the above Formula I and Formula II alkyl groups useful therein include those having from about 1 to about 10 carbon atoms. Aryl groups in Formula I and Formula II include phenyl and substituted phenyl groups.

In one embodiment of the present invention the transition metal is Rh, and the metal complex is of the formula [Rh(PR²₃)ₙX]ₚ, wherein R² is an alkyl or aryl, X is a halide, n is 1, 2 or 3, and p is at least 1, or of the formula [Rh(PR²₃ )ₙY]ₚ, wherein R² is an alkyl or aryl, n is 1, 2 or 3, p is at least 1 and Y is a halide compound, preferably including BF₄, PF₆, ClO₄ or OCOCF₃.

In another embodiment the transition metal is Ru and the metal complex is of the formula [RuX₂(PR²₃)ₙ]ₚ wherein R² is an alkyl or aryl, X is a halide, n is 1, 2, 3 or 4; and p is at least 1 or of the formula [RuYX(PR₃²)ₙ]ₚ wherein R² is an alkyl or aryl and wherein n=3, p is at least 1, Y=H and X=Cl; n=3, Y=H and X=H; n=4, X=H, Y=H; or n=2, 3 or 4, p is at least 1 and X=Y=ClO₄, CF₃SO₃H, PF₆ or BF₄.

A suitable method for producing a Ru complex of the present invention involves refluxing a Ru salt, for example RuCl₃xH₂O with an excess of triphenylphosphine in alcohol to form the complex [Ru(P(C₆H₅)₃)₃Cl₂]_{p.}

A Rh complex of the present invention is also commercially available, or can be prepared by refluxing rhodium trichloride with triphenylphosphine in alcohol, typically methanol or ethanol.

The catalytic conversion of the present invention has the further economic advantage of being able to convert a salt of codeine or morphine, for example codeine hydrochloride or morphine hydrochloride, to hydrocodone or hydromorphone, respectively.

The reaction of the present invention may be accomplished by any conventional process. A suitable process includes dissolving the reactant of Formula I in a suitable solvent in a reaction vessel. Suitable solvents include but are not limited to alcohols, preferably primary and secondary lower alkyl alcohols. The reaction vessel is then flushed with an inert atmosphere, typically nitrogen. The catalyst is added and the reaction mixture is refluxed under the inert atmosphere until the conversion is essentially complete, typically at least about an hour. The reaction mixture is cooled and crystals of the product are collected. The product may be purified by recrystallization in a suitable solvent as is well known in the art, or by any other suitable conventional method of purification.

In an alternative embodiment a tertiary amine, for example triethylamine, is added to the reaction mixture when the preferred Ru complex catalyst is used. The tertiary amine reduces the formation of side products, primarily the alkaloid neopine, a potential side product in reactions of the present invention utilizing the preferred Ru catalyst.

In another alternative embodiment the alkaloid compound is added to the reactants that form the catalyst and the catalyst formation reaction takes place in the presence of the alkaloid. The ability to form the catalyst and subsequently accomplish the catalytic conversion in the same reaction vessel further enhances the economy of the reaction.

### Examples

### Example 1

Codeine, 50.00 g, was dissolved in 200 ml methanol in a three-neck flask at room temperature. The flask was equipped with a condenser and nitrogen. The flask was flushed with nitrogen for five minutes with one neck opened. The catalyst, 0.50g of RhCl(P(C₆H₅)₃)₃ was added to the solution. The flask was then flushed with nitrogen for another five minutes, and the open neck was closed. The reaction mixture was stirred under nitrogen and heated to reflux for four hours, then cooled to 0°C for 30 minutes. The resulting crystals were removed by filtration. The collected crystals were washed four times with 10ml cold methanol (5 °C), and dried in air for one hour yielding pale yellow crystals (41.50 g, yield 83%). The filtrate was pumped down to dryness to give 6.14 g yellow solid. The solid residue was dissolved in 40 ml refluxing methanol and cooled to 0°C for 30 minutes and filtered. The collected crystals were washed four times with 3 ml portions of cold methanol (5°C) and air dried for 2 hours yielding 3.41 g (6.8%) white crystals. HPLC analysis and NMR spectra confirm that the product is pure hydrocodone.

### Example 2

Morphine, 50.00 g, was suspended in 500 ml methanol in a three neck flask equipped with condenser and nitrogen input and outlet. After refluxing under nitrogen for five minutes, one neck of the flask was opened. A catalyst, 0.50g RhCl(P(C₆H₅)₃)₃ was added to the container. The opened neck was closed with a stopper. The reaction mixture was stirred under nitrogen and heated to reflux for 6 hours, cooled down to 0°C for 30 minutes, and filtered. The collected solid was washed four times with cold methanol (5°C) and dried in air for 20 minutes. The solid was kept under vacuum (15 mm Hg) at room temperature for 1 hour, yielding a white powder (38.83 g, yield 77.7%). The filtrate was distilled under nitrogen until only 200 ml of solution remained. It was cooled down to 0°C for 30 minutes and filtered, yielding a white powder. The product was washed twice with 20 ml each and then once with 10ml of cold methanol (5°C) dried in air for 40 minutes to yield 3.48 g of a white powder product. HPLC analysis and NMR spectra confirm product is pure hydromorphone.

### Example 3

Ruthenium dimer was prepared by refluxing 1 g RuCl₃xH₂O with 3 equivalents P(C₆H₅)₃ in EtOH (100 ml) overnight. The resulting catalyst, [Ru(P(C₆H₅)₃)₂Cl₂]₂ was obtained as a black precipitate after filtration, 63% yield.

### Example 4

The catalyst of Example 3 was reacted with codeine in MeOH in the presence of triethylamine in the ratios shown in the table below. The reaction mixtures were flushed with N₂ for 5 minutes, heated to reflux under N₂, cooled to 0°C and filtered. The recovered crystals were washed twice with 5 ml MeOH and air dried to yield white crystals.

**Table 1**

| **Starting Materials** | **[Ru(P(C₆H₅)₃)₂Cl₂l₂** | **MeOH** | **NEt₃** | **Reaction time** | **Products** | **Hydrocodone/ codeine/neopine (area % by HPLC)** |
|---|---|---|---|---|---|---|
| Codeine 1.0 g | 10 mg | 5 ml | 0.25 ml | 2 h | 0.85 g | 96.7/1.8/0.33 |
| Codeine 1.0 g | 10 mg | 5 ml | 0.25 ml | 3 h | 0.99 g | 94.7/0/0 |
| Codeine 2.5 g | 25 mg | 12.5 ml | 0.725 ml | 3 h | 1.58 g | 100/0/0 |
| Codeine 1 g/ Codeine. sulfate 0.05g | 10 mg | 5 ml | 0.25 ml | 3 h | 0.61 g | 97.4/1.0/0 |
| Codeine 1 g/ CodeineHCL 0.05g | 55 mg | 10 ml | 0 | 3 h | 1.17 g | 77.5/2.5/0.8 |
| Codeine HCl 1.1g | 100 mg | 10 ml | 0 | 15 h | 1.16 g | 81.5/11.6/0 |
| Morphine (recry) 1.0 g | 80 mg | 10 ml | 0.25 | 3 days | 1.08 g | Hydromorphone/ morphine = 88.6/6.1 |

### Example 5

Codeine, 5.0 g, and 0.117g catalyst, Ru(P(C₆Hₛ)₃)₃Cl₂, were dissolved in 25 ml EtOH. The mixture was flushed with nitrogen for 5 minutes. After refluxing under nitrogen for 2 hours the reaction mixture was cooled down to 0°C and filtered. The collected crystals were washed twice with 5 ml each MeOH and dried in air for 1 hour to yield white crystals, 70% yield hydrocodone.

### Example 6

Morphine, 1.0 g, and 80 mg [Ru(P(C₆H₅)₃)₂Cl₂]₂ was suspended in 10 ml MeOH. The reaction mixture was flushed with N₂ for 3 minutes, after which 0.25 ml triethylamine was added, and the mixture flushed with N₂ for another 3 minutes. The reaction mixture was heated to reflux with stirring under N₂ for 72 hours. The resulting solid was found to be hydromorphone.

### Example 7

Codeine, 5.09 g, 25.4 mg RuCl₃xH₂O and 95.9 mg P(C₆H₅)₃ were added to 25 ml ethanol in a three neck flask. The flask was flushed with N₂ for 5 minutes. The mixture was heated until reactants dissolved, and then refluxed, under nitrogen, overnight. The resulting solution was washed twice with 5 ml each MeOH and air dried to yield 3.31 g product. Analysis determined the presence of hydrocodone and Ru catalyst.

### Example 8

Codeine-HCl, 1.1 g and 0.1 g RuCl₂(P(C₆H₅)₃)₃ were stirred in 10 ml MeOH and flushed with N₂ for 5 minutes. The reaction mixture was refluxed overnight under N₂, cooled to room temperature and dried under vacuum, yielding 1.16 g brown solid containing hydrocodone.

From the foregoing description those skilled in the art will appreciate that economical and efficient methods for the catalytic conversion of codeine or morphine into hydrocodone or hydromorphone, respectively, are provided.

## Claims

1. A method comprising catalytically converting a compound of Formula I into a compound of Formula II utilizing at least one transition metal complex of a tertiary phosphine halide, 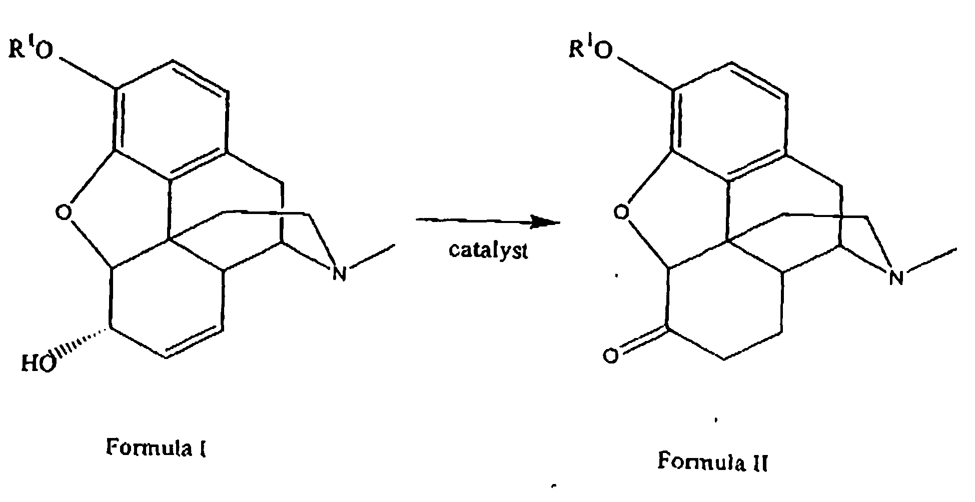 wherein R¹ is H, alkyl, aryl or acyl, and wherein the metal complex is of the formula (M(PR²₃)ₙXₘ]ₚ wherein M is a Group VIII-transition metal, R² is an alkyl or aryl, X is a halide or halide compound, n is 1, 2, 3 or 4, p is at least 1 and m is 1 or 2.

2. The method according to claim 1 for producing hydrocodone wherein Formula I is codeine and Formula II is hydrocodone.

3. The method according to claim 1 for producing hydrocodone comprising converting codeine into hydrocodone in the presence of at least one catalyst, wherein the catalyst includes RhCl(P(C₆H₅)₃)₃ or [Rᵤ(P(C₆H₅)₃)₂Cl₂]₂.

4. The method according to claim 1 for producing hydromorphone wherein Formula I is morphine and Formula II is hydromorphone

5. The method according to claim 1 for producing hydromorphone comprising converting morphine into hydromorphone in the presence of at least one catalyst, wherein the catalyst includes RhCl(P(C₆H₅)₃)₃ or [Ru(P(C₆H₅)₃)₂Cl₂]₂.

6. The method according to Claim 1 wherein R¹ is H or CH₃

7. The method of any one of Claims 1, 2 or 4 wherein the metal complex is of the formula [Rh(PR²₃)ₙX]ₚ wherein R² is an alkyl or aryl, X is a halide or halide compound, n is 1,2 or 3 and p is at least 1.

8. The method of any one of Claims 1, 2 or 4 wherein the metal complex is of the formula [Rh(PR²₃)ₙY]ₚ, wherein n is 1, 2 or 3, p is at least 1 and Y is selected from the group consisting of BF₄, PF₆, ClO₄ and OCOCF₃.

9. The method of any one of Claims 1, 2 or 4 wherein the metal complex is of the formula (RuX₂(PR²₃)ₙ]ₚ wherein R² is an alkyl or aryl, X is a halide, n is 1, 2, 3 or 4 and p is at least 1.

10. The method of any one of Claims 1, 2 or 4 wherein the metal complex is of the formula [RuYX(PR²₃)ₙ]ₚ wherein R² is an alkyl or aryl and wherein n=3, Y=H and X=Cl; or n-3, Y=H and X=H; or n-4, X=H, Y=H; or n=2, 3 or 4 and X=Y is selected from the group consisting of ClO₄, CF₃CO₂, PF₆ and BF₄.

## Patentansprüche

1. Verfahren, umfassend das katalytische Überführen einer Verbindung nach Formel I zu einer Verbindung nach Formel II unter Verwendung mindestens eines Übergangsmetallkomplexes eines tertiären Phosphinhalogenids, 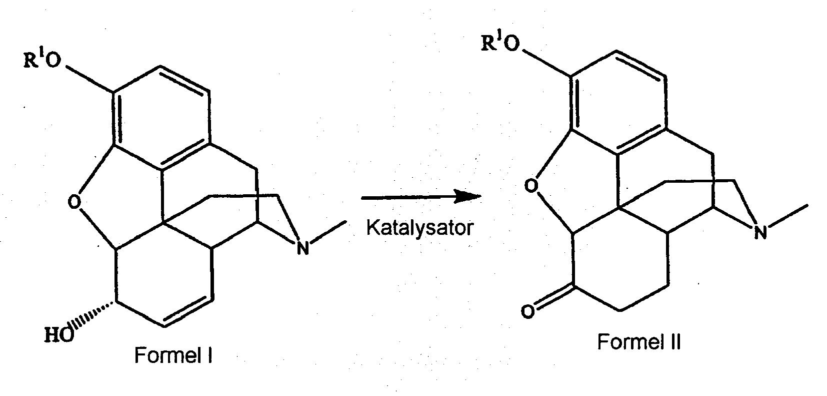 wobei R¹ gleich H, Alkyl, Aryl oder Acyl ist; und wobei der Überhangsmetallkomplex die Formel [M(PR²₃)ₙXₘ]ₚ aufweist; worin M ein Gruppe VIII-Übergangsmetall ist; R² ein Alkyl oder Aryl ist; X ein Halogen oder eine Halogenverbindung ist; n gleich 1, 2, 3 oder 4 ist; p mindestens 1 ist; und m gleich 1 oder 2 ist.

2. Verfahren nach Anspruch 1 zur Herstellung von Hydrocodon, wobei Formel I Codein ist und Formel II Hydrocodon ist.

3. Verfahren nach Anspruch 1 zur Herstellung von Hydrocodon, umfassend das Überführen von Codein zu Hydrocodon in der Gegenwart mindestens eines Katalysators, wobei der Katalysator RhCI(P(C₆H₅)₃)₃ oder [Ru(P(C₆H₅)₃)₂Cl₂]₂ umfasst.

4. Verfahren nach Anspruch 1 zur Herstellung von Hydromorphon, wobei Formel I Morphin ist und Formel II Hydromorphon ist.

5. Verfahren nach Anspruch 1 zur Herstellung von Hydromorphon, umfassend das Überführen von Morphin zu Hydromorphon in der Gegenwart mindestens eines Katalysators, wobei der Katalysator RhCl(P(C₆H₅)₃)₃ oder [Ru(P(C₆H₅)₃)₂Cl₂]₂ umfasst.

6. Verfahren nach Anspruch 1, wobei R¹ gleich H oder CH₃ ist.

7. Verfahren nach einem der Ansprüche 1, 2 oder 4, wobei der Metallkomplex die Formel [Rh(PR²₃)ₙX]ₚ aufweist; worin R² ein Alkyl oder Aryl ist; X ein Halogen oder eine Halogenverbindung ist; n gleich 1, 2 oder 3 ist; und p mindestens 1 ist.

8. Verfahren nach einem der Ansprüche 1, 2 oder 4, wobei der Metallkomplex die Formel [Rh(PR²₃)ₙY]ₚ aufweist; worin n gleich 1, 2 oder 3 ist; p mindestens 1 ist; und Y ausgewählt ist aus der Gruppe, bestehend aus BF₄, PF₆, ClO₄ und OCOCF₃.

9. Verfahren nach einem der Ansprüche 1, 2 oder 4, wobei der Metallkomplex die Formel [RuX₂(PR²₃)ₙ]ₚ aufweist; worin R² ein Alkyl oder Aryl ist; X ein Halogen ist; n gleich 1, 2, 3 oder 4 ist; und p mindestens 1 ist.

10. Verfahren nach einem der Ansprüche 1, 2 oder 4, wobei der Metallkomplex die Formel [RuYX(PR²₃)ₙ]ₚ aufweist; worin R² ein Alkyl oder Aryl ist; und wobei n=3, Y=H und X=Cl; oder n=3, Y=H und X=H; oder n=4, X=H, Y=H; oder n=2, 3 oder 4, und X=Y ausgewählt ist aus der Gruppe, bestehend aus ClO₄, CF₃CO₂, PF₆ und BF₄.

## Revendications

1. Procédé comprenant la conversion catalytique d'un composé de formule I en un composé de formule II en utilisant au moins un complexe de métal de transition d'un halogénure de phosphine tertiaire, 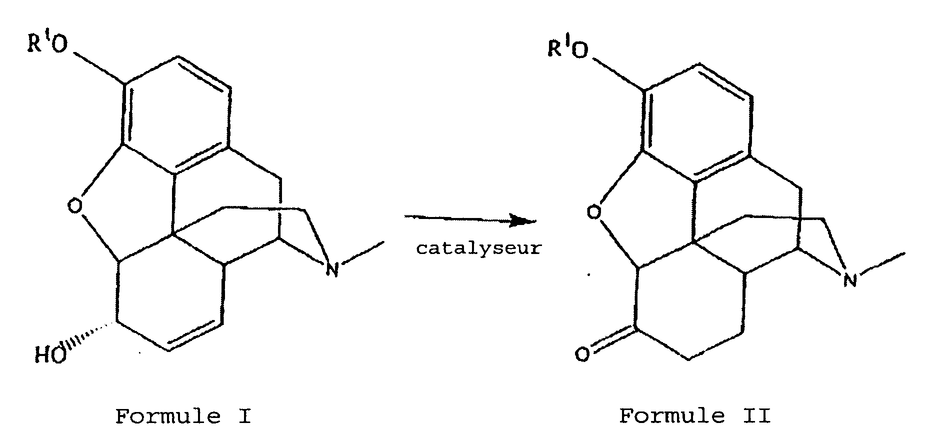 où R¹ représente H, un groupe alkyle, aryle ou acyle, et où le complexe métallique répond à la formule [M(PR²₃)ₙXₘ]ₚ dans laquelle M représente un métal de transition du Groupe VIII, R² représente un groupe alkyle ou aryle, X représente un halogénure ou composé halogéné, n est égal à 1, 2, 3 ou 4, p est égal à au moins 1 et m est égal à 1 ou 2.

2. Procédé suivant la revendication 1, pour la production d'hydrocodone, dans lequel la formule I représente la codéine et la formule II représente l'hydrocodone.

3. Procédé suivant la revendication 1 pour la production d'hydrocodone, comprenant la conversion de codéine en hydrocodone en présence d'au moins un catalyseur, dans lequel le catalyseur comprend RhCl(P(C₆H₅)₃)₃ ou [Ru(P(C₆H₅)₃)₂Cl₂]₂.

4. Procédé suivant la revendication 1, pour la production d'hydromorphone, dans lequel la formule I représente la morphine et la formule II représente l'hydromorphone.

5. Procédé suivant la revendication 1 pour la production d'hydromorphone, comprenant la conversion de morphine en hydromorphone en présence d'au moins un catalyseur, dans lequel le catalyseur comprend RhCl (P (C₆H₅) ₃) ₃ ou [Ru(P(C₆H₅)₃)₂Cl₂]₂.

6. Procédé suivant la revendication 1, dans lequel R¹ représente H ou un groupe CH₃.

7. Procédé suivant l'une quelconque des revendications 1, 2 et 4, dans lequel le complexe métallique répond à la formule [Rh(PR²₃)ₙX]ₚ dans laquelle R² représente un groupe alkyle ou aryle, X représente un halogénure ou composé halogéné, n est égal à 1, 2 ou 3 et p est égal à au moins 1.

8. Procédé suivant l'une quelconque des revendications 1, 2 et 4, dans lequel le complexe métallique répond à la formule [Rh(PR²₃)ₙY]ₚ dans laquelle n est égal à 1, 2 ou 3, p est égal à au moins 1 et Y est choisi dans le groupe consistant en BF₄, PF₆, C10₄ et OCOCF₃.

9. Procédé suivant l'une quelconque des revendications 1, 2 et 4, dans lequel le complexe métallique répond à la formule [RuX₂(PR²₃)ₙ]ₚ dans laquelle R² représente un groupe alkyle ou aryle, X représente un halogénure, n est égal à 1, 2, 3 ou 4 et p est égal à au moins 1.

10. Procédé suivant l'une quelconque des revendications 1, 2 et 4, dans lequel le complexe métallique répond à la formule [RuYX(PR²₃)ₙ]ₚ dans laquelle R² représente un groupe alkyle ou aryle, et dans laquelle n = 3, Y = H et X = Cl ; ou n = 3, Y = H et X = H ; ou n = 4, X = H, Y = H, ou bien n = 2, 3 ou 4 et X = Y et est choisi dans le groupe consistant en des groupes ClO₄, CF₃CO₂, PF₆ et BF₄.
